(19) Europäisches Patentamt
European Patent Office
Office européen des brevets

(11) Publication number: **0 429 895 A1**

# (12) EUROPEAN PATENT APPLICATION

(21) Application number: **90121179.7**

(51) Int. Cl.[5]: **C07D 501/59**, //A61K31/545

(22) Date of filing: **06.11.90**

(30) Priority: **01.12.89 IT 2257589**

(43) Date of publication of application:
**05.06.91 Bulletin 91/23**

(84) Designated Contracting States:
**AT BE CH DE DK ES FR GB GR IT LI LU NL SE**
**Bulletin**

(71) Applicant: **IRCA S.P.A. - INDUSTRIE RICERCHE CHIMICHE D' ALBANO**
**Via Tonale 87**
**I-24061 Albano S'Alessandro, Bergamo(IT)**

(72) Inventor: **Ambrosini, Leonardo**
**Via Sant'Antonino 7/A**
**I-24100 Bergamo(IT)**

(74) Representative: **Giambrocono, Alfonso, Dr. Ing. et al**
**Ing. A. Giambrocono & C. S.r.l. Via Rosolino Pilo 19/B**
**I-20129 Milano(IT)**

## (54) Process for preparing monohydrate 7-(D-2-amino-2-phenylacetamido)-3-cefem-4-carboxylic acid and intermediate formed in the process.

(57) Process for preparing monhydrate 7-(D-2-amino-phenylacetamido)-3-chloro-3-cefem-4-carboxilic acid, comprising the condensation between silanized 7-amino-3-chloro-cephalosporanic acid and D(-)-phenylglycine chloride hydrochloride in the presence of a proton acceptor, such condensation yields a product which precipitates in form of solvate of acetonitrile, from which the final product is obtained by known techniques, said process allowing to obtain higher yields and a purer product.

EP 0 429 895 A1

The present invention relates to a new process for preparing monohydrate 7-(D-2-amino-2-phenylacetamido)-3--chloro-3-cefem-4-carboxylic acid (cefaclor) and to an intermediate of said process.

Cefaclor is a molecule belonging to the group of cephalosporines, known antibiotics widely employed; in this group, it holds a particular position owing to its chemical structure:

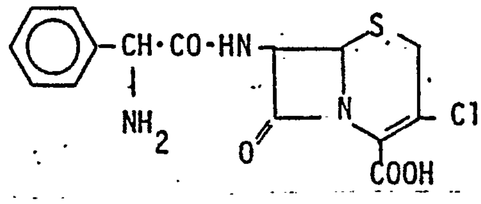

which, because of chloro atom in 3-position, provides the compound with a remarkable metabolic stability.

Cefaclor is employed with very good results in the clinical practice, and it has been widely described in literature for what concerning its synthesis, configuration and therapeutical activity (U.S. patent 3,925,372); J. Med. Chem. 1975, 18-4, 403-8; Anal. Profiles Drug. Subst., 1980, 9, 107-23).

The methods of syntesis of cefaclor emply the condensation of a derivative of 7-amino-3-chloro-cephalosporanic acid of formula (I) with a derivative of D(-)--phenylglycine of formula (II), according to the scheme shown below:

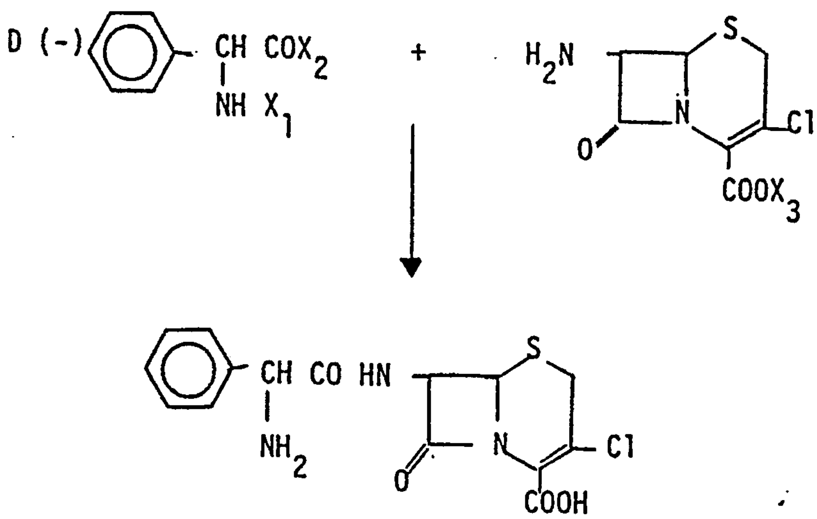

This kind of reaction is carried out according to a scheme common in the synthesis of peptides and many cephalosporines. Generally the amino group of phenylglycine (II) is protected by a Dane's salt or simply by salification with hydrochloric acid ($X_1$), while the carboxy group is activated as chloride or mixed anhydride ($X_2$): in the latter case the anhydride obtained from pivaloylchloride or from methyl chlorocarbonate is often employed. On the other side the 7-amino-3-chloro-cephalosporanic acid (I) is protected on carboxyl group by silanization or by esterification or salification ($X_3$). Employing said procedures, the yields up to now obtained do not exceed 45% (U.S. patent 3,925,372).

The silanization of 7-amino-3-chloro-cephalosporanic acid shows the double advantage of protecting the carboxy group and of making the compound soluble. It is effected by means of a silanizating agent, preferably in the presence of a coadjuvant of silanization, in a suitable organic solvent. The experiments of the present inventors have shown useful as silanizating agents the following compounds: trimethylchlorosilane, hexamethylsilarane, dimethylchlorosilane, tri methylsilylacetsmide, bis-trimethylsilylacetamide, bis-trimethylsilylurea and trimethylsilyldiethylamino. As useful co-adjuvants of silanization the following compounds have been employed: triethylamine, diethylamine and, in general, all the tertiary amines like pyridine, picoline, lutidine, dimethylaminopyridine, pyrazolidine, piperidine, morpholine, methylmorpholine. The organic solvent is usually selected from the group comprising dimethylformamide, pyridine, dimethyl

sulfoxide, tetrahydrofuran, acetone, methylene chloride, 1,2-dichloroethane, 1,1,2-trichloroethane, 1,1,1--trichloroethane, chloroform, 1,1-dichloroethane and aceto-nitrile.

Taking the industrial production of cefaclor into consideration, it is noted that the condensation reaction shown above is the step decisive for the cost, also because, the technology connected with the production of 7-amino-3--chloro-cephalosporanic acid being very complex, the price of this intermediate is very high. Hence a method for synthesizing cefaclor providing higher yields became desirable, also in view of the remarkable cost of the reagent.

It has been now surprisingly found a method of synthesis of cefaclor which significantly reduces the industrial cost while increasing yields. A further feature of the present invention resides in an intermediate of the synthesis which allows to obtain the final product with a high degree of purity.

In fact it has been surprisingly found that effecting the condensation between D(-)-phenylglycine chloride hydrochloride and silanized 7-amino-3-chloro-cephalosporanic acid in the presence of a proton acceptor, reaction yields also higher than 70%, anyway remarkably higher than the ones of prior art, are easy to obtain.

The function of this proton acceptor within the scope of the condensation between the reactants is twofold: on one hand it is able to capture the hydrochloric acid getting loose during said phase (and which can degrade the final product causing a consequent decrease of the yields); on the other hand it probably behaves as a catalyst by reaoting with the silanizating agent. This second feature of the utility of the proton acceptor has not been yet perfectly clairified, anyway it does not affect the range of protection of the present invention.

The proton acceptor is chosen in the group consisting of acetamide, dimethylacetamide, pyridine, dimethylaminopyridine, methylmorpholine, nicotinamide and other derivatives of nicotinic acid. In the present invention the preferred proton acceptor is acetamide. It is employed at least in stoichiometric amount with respect to D(-)-phenylglycine chloride hydrochloride, and preferably in a molar ratio of about 2.5:1.

The condensation is effectively carried out at a temperature varying from -30$^\circ$C to +50$^\circ$C, preferably at -10$^\circ$C.

A further feature of the present invention relates to the obtainment of a new cefaclor solvate which allows to readily obtain a final product with a high degree of purity, around 98%.

After the above condensation reaction, for isolating and purifying the obtained product, water and acetonitrile are added, and pH is adjusted to the isoelectric one of the product by means of an organic base suitable for the present solvents. In this way the precipitation of the addition product (solvate) of cefaclor and acetonitrile takes place and in such product the constituents are in equimolar ratio. If acetonitrile has been already used as solvent in the condensation, it can directly cause the precipitation of the solvate, providing that its residual amount from condensation is sufficient for such scope. Cefaclor is readily got loose from said solvate intermediate by adding a mixture of water and a water-miscible solvent, for example lower alcohols or acetone, according to an already known technique.

For better illustrating the present invention, some examples are herein provided which anyway have not to be considered as limitating the herein described process.

EXAMPLE 1

23.46 g (0.1 mole) of 7-amino-3-chloro-cephalosporanic acid are admixed with 260 g of methylene chloride. After distilling off about 13 g of solvent for making the environment anhydrous the reaction mixture is cooled at 20$^\circ$C, and then 25 g (0.23 mole) of trimethylchlorosilane are added therein. The reaction mixture is cooled at -5$^\circ$C and after that 19.2 g (0.19 mole) of triethylamine are added during 1.5 hours, while keeping the temperature at 0$^\circ$C. The reaction mixture is then refluxed for 2 hours.

At a temperature of about 0$^\circ$C 135 ml of water are slowly added, the phases are separated and the acqueous layer is collected and filtered on charcoal. 130 ml of acetonitrile are added therein, and pH is adjusted at 5.2 by means of addition of triethylamine. The precipitate is collected on filter and washed with a mixture 1:2 acetonitrile:acetone.

The so-obtained solvate is added to 110 ml of water warmed at 56$^\circ$C, and the mixture is maintained at such temperature for 15 minutes. Then 70 g of acetone are added, and at last the mixture is cooled at 10$^\circ$C. After two hours under stirring, the precipitate is filtered and dried. In this way 27.5 g of cefaclor are obtained.

| | |
|---|---|
| Yield: | 71% |
| Title (HPLC) of anhydrous: | 98.2% |
| K.F.: | 4.68% |

EXAMPLE 2

Employing the same amounts and conditions stated in Example 1, but adding acetamide before phenylglycine chloride hydrochloride, 24.6 g of cefaclor are obtained.

| | |
|---|---|
| Yield: | 63.9 |
| Title (HPLC) of anhydrous: | 98.01% |
| K.F.: | 6.36% |

EXAMPLE 3

Employing the same amounts and conditions stated in Example 1, but adding 5.7 g of dimethylacetamide instead of 14.2 g of acetamide, 22.5 g of cefaclor are obtained.

| | |
|---|---|
| Yield: | 58.3% |
| Title (HPLC) of anhydrous: | 97.5% |
| K.F.: | 6.18% |

## Claims

1. Process for preparing monohydrate 7-(D-2-amino-2--phenylacetamido)-3-chloro-3-cefem-4-carboxylic acid obtained from condensation between silanized 7-amino-3-chloro-cephalosporanic acid and D(-)-phenylglycine chloride hydrochloride and sequentially purified by treatment with a mixture of water and a water-miscible solvent, characterized in that the condensation between silanized 7-amino-3-chloro-cephalsporanic acid and D(-)-phenylglycine chloride hydrochloride takes place in the presence of a proton acceptor selected from the group consisting of acetamide, dimethylacetamide, pyridine, dimethylaminopyridine, methylmorpholine, nicotinamide and derivatives of nicotinic acid, the production of condensation is precipitated by acetonitrile, in the presence of water, in form of the relevant solvate of acetonitrile, from which monohydrate 7-(D-2-amino-2-phenylacetamido)-3-chloro-3-cefem-4-carboxylic acid is purified as above said.

2. Process according to claim 1, wherein the proton acceptor is added after addition of D(-)-phenylglycine chloride hydrochloride.

3. Process according to claim 1 and 2, wherein the proton acceptor is acetamide.

4. Process according to claims from 1 to 3, wherein the proton acceptor is employed in at least stoichiometric amount with respect to D(-)-phenylglycine chloride hydrochloride.

5. Process according to claim 4, wherein the proton acceptor is employed in a molar ratio of 2.5:1 with respect to D(-)phenylglycine chloride hydrochloride.

**6.** Process according to any of the precedent claims, wherein the temperature of condensation ranges between -30 °C and +50 °C.

**7.** Process according to claim 6, wherein the temperature of condensation is -10 °C.

**8.** Monohydrate 7-(D-2-amino-2-phenylacetamido)-3-chloro--3-cefem-4-carboxylic acid solvate of acetonitrile, wherein the molar ratio between the two components is 1:1.

European Patent Office

# EUROPEAN SEARCH REPORT

Application Number

EP 90121179.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | US - A - 3 925 372 (R.R. CHAUVETTE) * Example 18 * -- | 1-8 | C 07 D 501/59 //A 61 K 31/545 |
| A | EP - A2 - 0 090 240 (BAYER AG) * Claims; examples * ---- | 1-8 | |
| | | | TECHNICAL FIELDS SEARCHED (Int. Cl.5) |
| | | | C 07 D 501/00 |

The present search report has been drawn up for all claims

| Place of search | Date of completion of the search | Examiner |
|---|---|---|
| VIENNA | 12-02-1991 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)